# FASCICULE DE BREVET EUROPEEN

(11) **EP 3 705 192 B1**
(45) Date de publication et mention de la délivrance du brevet: **07.07.2021**
(21) Numéro de dépôt: 20158423.2
(22) Date de dépôt: 20.02.2020
(51) Int. Cl.: B05D 3/12, B05D 3/00, B05D 3/04, B05D 7/22, B05D 7/14, A61M 5/32, C03C 17/00, C03C 17/22, B05D 3/10

(54) **PROCÉDÉ POUR APPLIQUER UN REVÊTEMENT FLUORÉ ET/OU CÉRAMIQUE SUR UNE PAROI INTERNE D'UN TUBE DE FAIBLE DIAMÈTRE, TEL QU'UNE AIGUILLE DE SERINGUE**
VERFAHREN ZUM AUFBRINGEN EINER FLUORIERTEN UND/ODER KERAMISCHEN BESCHICHTUNG AUF EINE INNENWAND EINES RÖHRCHENS MIT GERINGEM DURCHMESSER, WIE ETWA EINE SPRITZENNADEL
METHOD FOR APPLYING A FLUORINATED AND/OR CERAMIC COATING TO AN INTERNAL WALL OF A LOW DIAMETER TUBE, SUCH AS A SYRINGE NEEDLE

(30) Priorité: 02.03.2019 FR 1902173
(43) Date de publication de la demande: 09.09.2020
(73) Titulaire: Slupecki, Annie, 45000 Orléans (FR); Slupecki, Patrice, 45000 Orléans (FR)
(72) Inventeur: Slupecki, Annie, 45000 Orléans (FR); Slupecki, Patrice, 45000 Orléans (FR)
(74) Mandataire: Lequien, Philippe

(56) Documents cités:
- US-A1- 2005 255 240
- US-A1- 2012 164 325
- US-A1- 2012 315 381

## Description

Le domaine de l'invention est celui des techniques de circulation fluidique à l'intérieur de tube de petit et faible diamètres.

Plus précisément, l'invention concerne une technique pour appliquer un revêtement de préférence mis non exclusivement alimentaire, en particulier du type fluoré et/ou céramique, sur une paroi interne d'un tube de faible diamètre, tel qu'une aiguille.

L'invention s'applique en particulier à des tubes dont la paroi est opaque ou transparente, fait d'un matériau pouvant supporter une température supérieure à 100°C.

En outre, le procédé peut être utilisé sur des tubes droits, courbés ou hélicoïdaux, s'agissant d'une aiguille de seringue, de prélèvement, ou un tube d'injection, ou encore la partie interne d'un piston.

Dans le domaine de l'invention, l'application d'un revêtement alimentaire, fluoré et/ou céramique, à l'intérieur d'un corps de petit diamètre pose des difficultés majeures lorsqu'il s'agit, à une échelle industrielle, d'obtenir un revêtement uniforme notamment quant à son épaisseur et sa régularité. Ces difficultés tendent à atteindre encore un niveau supérieur lorsqu'il s'agit de réaliser un revêtement uniforme devant de plus être obtenu avec une très bonne accroche et avec une qualité qui soit durable.

US 2012/315381 A1 décrit un procédé pour appliquer un revêtement céramique sur un paroi interne des canaux de faible diamètre, comprenant les étapes de: aspiration et écoulement dans les tubes, vidange du liquide contenu dans les tubes, à l'issue de laquelle un dépôt de revêtement sur la paroi interne des tubes est obtenu; dessolvatation du revêtement par circulation d'air forcée et cuisson du revêtement à une température comprise entre 150°C et 450°C. En outre, le document mentionne une attaque chimique des parois interne avec des acides ou alcalins.

US 2012/164325 A1 fait état d'un procédé de revêtement de la surface intérieure d'une buse d'alimentation mince d'un diamètre compris entre 0.05 et 0.4 mm qui est appliquée sur la surface intérieure. Ledit processus comprenant les étapes d'aspiration dans le tube liquide fluoré, vidange du liquide contenu dans les tubes, à l'issue de laquelle un dépôt de revêtement sur la paroi interne des tubes est obtenu. If

US 2005/255240 A1 divulgue un procédé de revêtement de la surface intérieure d'un tuyau d'un diamètre compris entre 0.2 et 5 mm qui est appliquée sur la surface intérieure. Ledit processus comprenant les étapes d'aspiration dans le tube liquide fluoré, vidange du liquide contenu dans les tubes, à l'issue de laquelle un dépôt de revêtement sur la paroi interne des tubes est obtenu, dessolvatation du revêtement par évaporation.

Bien entendu, ces difficultés d'application du revêtement à l'intérieur du tube, et en particulier de l'aiguille, sont liées entre autres à son faible diamètre interne, s'agissant de réaliser le revêtement après fabrication du tube lui-même, avec des contraintes liées au passage à l'intérieur du tube.

Il est à noter que, non seulement dans le domaine de l'alimentaire, mais également dans le domaine du médical, pharmaceutique ou du cosmétique, il est exigé que toute application d'un revêtement interne soit réalisée avec un revêtement de type alimentaire.

L'invention a notamment pour objectif de surmonter les difficultés de l'art antérieur.

Plus précisément, l'invention a pour objectif de proposer une technique d'application d'un revêtement de type alimentaire ou non à l'intérieur d'un tube de faible diamètre interne, en métal, inox verre ou équivalent.

L'invention a également pour objectif de réaliser un tel revêtement interne avec une bonne accroche et une bonne tenue dans le temps.

Ces objectifs, ainsi que d'autres qui apparaîtront par la suite, sont atteints grâce à l'invention qui a pour objet un procédé pour appliquer un revêtement coloré fluoré et/ou céramique sur une paroi interne d'un tube de faible diamètre tel qu'une aiguille de seringue, comprenant les étapes de :
- aspiration et/ou écoulement dans le tube d'un liquide contenant le revêtement ;
- vidange du liquide contenu dans le tube, à l'issue de laquelle un dépôt de revêtement sur la paroi interne du tube est obtenu ;
- dessolvatation du revêtement par circulation d'air forcée ;
- cuisson du revêtement ;
caractérisé en ce qu'il comprend une étape de préparation de la surface interne réalisée par attaque mécanique, l'étape de préparation de la surface interne étant réalisée à l'aide d'un fil introduit dans le tube interne et animé d'un mouvement de va-et-vient contre la paroi interne du tube.

Ainsi, grâce à l'invention, on obtient un procédé qui permet d'appliquer un revêtement alimentaire ou non, en particulier fluoré et/ou céramique, sur la paroi interne d'un tube présentant une faible diamètre interne tel une aiguille de seringue, qui permet d'obtenir un revêtement uniforme, en particulier dans son épaisseur.

L'étape de préparation de la surface interne du tube permet de générer des conditions optimales d'accroche du revêtement sur la paroi interne du tube, avec une bonne tenue dans le temps.

Comme indiqué par la suite, la préparation de l'intérieur du tube peut être réalisée :
- par voie chimique (liquide ou vapeur) et mécanique (érosion par un filin fin type tungstène diamant par exemple) ;
- ou par voie uniquement mécanique (érosion par un filin fin type tungstène diamant par exemple) ;
sur une durée pouvant aller de quelques minutes à plusieurs heures en fonction du matériau.

Selon un mode de réalisation particulier, l'étape d'aspiration et/ou d'écoulement est répétée à plusieurs reprises avant l'étape de vidange.

Avec la répétition de cette étape, on augmente le dépôt du revêtement contenu dans le liquide sur la paroi interne du tube.

Selon un mode de réalisation préféré, la circulation d'air forcée est réalisée à une température comprise entre la température ambiante et 150°C.

Le pilotage de la température, combiné préférentiellement à celui du débit et de la pression, conduit à une parfaite dessolvatation du revêtement.

On note que la dessolvatation du revêtement se traduit par un pré-séchage uniforme du revêtement, lui conférant une bonne tenue avant cuisson.

Préférentiellement, la circulation d'air forcée est réalisée en étuve.

Il est ainsi aisé de contrôler les conditions de température, et également de pression, dans lesquelles la circulation d'air forcée est réalisée.

Selon un mode de réalisation envisageable, la succession des étapes d'aspiration, de vidange, de dessolvatation et de cuisson est répétée.

Une telle répétition de la succession des étapes d'aspiration, de vidange, de dessolvatation et de cuisson sera mise en œuvre en fonction de la nature du revêtement à appliquer.

Selon un mode de réalisation préférentiel, lors de l'étape de préparation de la surface interne, le fil introduit dans le tube interne est un filin du type tungstène diamant.

Selon une solution l'étape de préparation de la surface interne est également réalisée par attaque chimique.

Selon un premier mode de réalisation, l'attaque chimique de l'étape de préparation de la surface interne est réalisée par aspersion interne de liquide ou injection de vapeur.

Selon un deuxième mode de réalisation, l'étape de préparation de la surface interne est également réalisée par sablage.

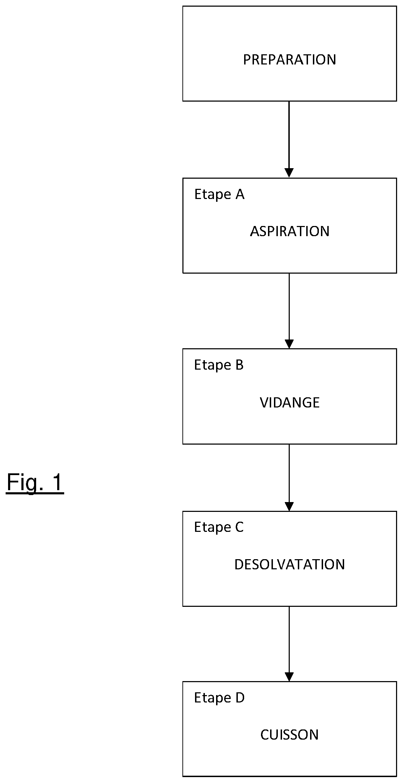

Avantageusement, la cuisson du revêtement est réalisée à une température comprise entre 150°C et 450°C.
- D'autres caractéristiques et avantages de l'invention apparaîtront plus clairement à la lecture de la description suivante d'un mode de réalisation préférentiel de l'invention, donné à titre d'exemple illustratif et non limitatif, et de :la figure 1 unique qui procure une représentation synoptique d'un procédé selon l'invention.

En référence à la figure 1, le procédé selon l'invention vise à appliquer un revêtement fluoré et/ou céramique sur la paroi interne d'un tube, et plus spécifiquement sur la paroi interne d'une aiguille de prélèvement ou d'injection. Le procédé peut être applique sur des aiguilles d'injection, autrement dit des aiguilles de seringue, présentant un diamètre interne de 0,2 mm pour les plus fines, et pouvant aller jusqu'à plusieurs centimètres

Selon le principe de l'invention, le procédé pour appliquer un revêtement fluoré et/ou céramique sur la paroi interne du tube (ou de l'aiguille) comprend une étape d'application du revêtement sur la surface interne du récipient (étape A). Le dépôt du revêtement est obtenu en aspirant ou par écoulement dans le tube (ou l'aiguille) d'un liquide contenant le revêtement, c'est-à-dire un liquide dans lequel est dissout un composant destiné à se déposer sur la paroi interne pour former sur celle-ci un revêtement.

L'aspiration du liquide contenant le revêtement peut être effectuée à l'aide d'une seringue. Cette étape peut être répétée à plusieurs reprises, en procédant à plusieurs allers-retours du piston de la seringue.

Après l'étape A, répétée ou non, le liquide contenant le revêtement est vidangé du corps du tube (étape B). A l'issue de l'étape B, un dépôt de revêtement sur la paroi interne du tube est formé, avec une épaisseur plus ou moins importante selon la répétition ou non de l'étape A.

Il est ensuite procédé à une étape de dessolvatation du revêtement par circulation d'air forcée et/ou par gravité dans le tube (étape C).

Pour cela, le tube est raccordé à ses deux extrémités à un circuit de circulation d'air couplé à un dispositif de type compresseur par exemple.

Préférentiellement, cette étape est conduite dans une enceinte pourvue de moyens de réglage de la température et de la pression. Cette enceinte est avantageusement une étuve.

Selon une caractéristique de l'invention, l'étape de circulation d'air forcée est conduite à une température comprise entre la température ambiante 50°C et 150°C. L'étuve utilisée le cas échéant est donc paramétrée pour engendrer de telles conditions de température.

De plus, le dispositif générant la circulation d'air forcée dans le tube est conçu pour pouvoir être disposé dans une étuve et, bien entendu, pour fonctionner à l'intérieur de l'étuve. En pratique, ledit dispositif comprend une pluralité de circuits de circulation d'air, chacun destiné à être accordé à un tube. Ainsi, il est possible de procéder à une étape de dessolvatation du revêtement d'un grand nombre de tubes simultanément.

De façon à améliorer encore la solvatation du revêtement, on reproduit l'étape de circulation d'air forcée en inversant le sens de circulation d'air. Pour cela, on inverse le sens de passage de la circulation d'air.

Le temps de circulation d'air est ajusté en fonction de la nature du revêtement et de son épaisseur.

Toujours en fonction du type de revêtement et l'épaisseur souhaité de celui-ci, il est possible à ce stade de recommencer les étapes du procédé à partir de l'étape A.

Après dessolvatation du revêtement, on procède à une étape de cuisson du revêtement, conduite à une température comprise entre 150°C et 450°C. La température de cuisson peut être constante ou progressive, avec ou sans paliers, et est adaptée en fonction de la nature et de l'épaisseur du revêtement.

Une étape en amont du procédé selon l'invention est avantageusement réalisée, s'agissant de préparer la surface interne du tube (ou de l'aiguille) par une attaque chimique et mécanique, ou par une attaque mécanique uniquement.

Selon le principe de l'invention, l'étape de préparation de la surface interne est réalisée à l'aide d'un fil introduit dans le tube interne, de tel sorte que le fil vienne frotter la paroi interne de telle sorte à provoquer une attaque abrasive. Pour cela, le fil peut être animé d'un mouvement de va-et-vient contre la paroi interne du tube.

Complémentairement à l'attaque mécanique à l'aide d'un fil, l'étape de préparation peut également intégrer les variantes suivantes :
Ainsi, selon une première variante de réalisation, l'étape de préparation de la surface interne consiste également en une attaque chimique obtenue par aspersion ou écoulement d'un liquide ou injection de vapeur à l'intérieur du tube.

Selon une deuxième variante de réalisation, l'étape de préparation de la surface interne est réalisée par une attaque mécanique consistant également à opérer un sablage de la paroi interne du tube. Ce type d'attaque peut être effectué en utilisant un sable spécial.

Selon une troisième variante la combinaison de plusieurs des techniques citées ci-dessus peut être réalisée.

Ainsi, la combinaison des phases de préparation de la surface interne du tube, d'application du revêtement et des phases post-application permet d'obtenir une application du revêtement avec une accroche de très bonne qualité, conduisant à une très bonne tenue dans le temps.

Globalement, avec le procédé selon l'invention qui vient d'être décrit, on obtient les avantages suivants :
- la circulation d'air forcée à l'intérieur du récipient, permettant la dessolvatation et/ou la cuisson du revêtement appliqué sur la surface interne ou sur le bord du récipient, est réalisé tandis que la paroi du récipient est chauffée (par la température à l'intérieur d'une étuve, ou par tout autre système de chauffage), ce qui évite d'éventuels phénomènes de condensation particulièrement nuisibles pour l'uniformité et la bonne tenue d'un revêtement alimentaire tel qu'un revêtement fluoré ;
- il est possible de réaliser la dessolvatation d'une pluralité de récipients simultanément.

## Revendications

1. Procédé pour appliquer un revêtement coloré fluoré et/ou céramique sur une paroi interne d'un tube de faible diamètre tel qu'une aiguille de seringue, comprenant les étapes de :
- aspiration et/ou écoulement dans le tube d'un liquide contenant le revêtement ;
- vidange du liquide contenu dans le tube, à l'issue de laquelle un dépôt de revêtement sur la paroi interne du tube est obtenu ;
- dessolvatation du revêtement par circulation d'air forcée ;
- cuisson du revêtement ;
**caractérisé en ce qu'**il comprend une étape de préparation de la surface interne réalisée par attaque mécanique, l'étape de préparation de la surface interne étant réalisée à l'aide d'un fil introduit dans le tube interne et animé d'un mouvement de va-et-vient contre la paroi interne du tube.

2. Procédé selon la revendication 1, **caractérisé en ce que** l'étape d'aspiration est répétée à plusieurs reprises avant l'étape de vidange.

3. Procédé selon la revendication 1, **caractérisé en ce que** la circulation d'air forcée est réalisée à une température comprise entre l'air ambiant et 150°C.

4. Procédé selon la revendication 1, **caractérisé en ce que** la circulation d'air forcée est réalisée en étuve.

5. Procédé selon l'une quelconque des revendications 1 à 3, **caractérisé en ce que** la succession des étapes d'aspiration, de vidange, de dessolvatation et de cuisson est répétée.

6. Procédé selon l'une quelconque des revendications précédentes, **caractérisé en ce que**, lors de l'étape de préparation de la surface interne, le fil introduit dans le tube interne est un filin du type tungstène diamant.

7. Procédé selon l'une quelconque des revendications précédentes, **caractérisé en ce que** l'étape de préparation de la surface interne est également réalisée par attaque chimique.

8. Procédé selon la revendication précédente, **caractérisé en ce que** l'attaque chimique de l'étape de préparation de la surface interne est réalisée par aspersion interne de liquide ou injection de vapeur.

9. Procédé selon l'une quelconque des revendications précédentes, **caractérisé en ce que** la cuisson du revêtement est réalisée à une température comprise entre 150°C et 450°C.

## Patentansprüche

1. Verfahren zum Aufbringen einer farbigen fluorierten und/ oder keramischen Beschichtung auf eine Innenwand eines Röhrchens mit geringem Durchmesser, wie etwa eine Spritzennadel, die folgenden Schritte umfassend:
- Absaugen und/ oder Strömen lassen einer die Beschichtung enthaltenden Flüssigkeit in dem Röhrchen;
- Entleeren der in dem Röhrchen enthaltenen Flüssigkeit, an dessen Ende eine Beschichtungsablagerung an der Innenwand des Röhrchens erhalten wird;
- Desolvieren der Beschichtung durch erzwungene Luftzirkulation;
- Brennen der Beschichtung;
**dadurch gekennzeichnet, dass** es einen Vorbereitungsschritt der Innenoberfläche umfasst, der durch mechanischen Angriff realisiert wird, wobei der Vorbereitungsschritt der Innenoberfläche mithilfe eines Drahtes realisiert wird, der in das innere Röhrchen eingeführt, und an der Innenwand des Röhrchens in Hin- und Herbewegung versetzt wird.

2. Verfahren nach Anspruch 1, **dadurch gekennzeichnet, dass** der Schritt des Absaugens vor dem Schritt des Entleerens mehrmals wiederholt wird.

3. Verfahren nach Anspruch 1, **dadurch gekennzeichnet, dass** die erzwungene Luftzirkulation bei einer Temperatur realisiert wird, die zwischen der Umgebungsluft und 150°C enthalten ist.

4. Verfahren nach Anspruch 1, **dadurch gekennzeichnet, dass** die erzwungene Luftzirkulation im Trockenofen realisiert wird.

5. Verfahren nach einem der Ansprüche 1 bis 3, **dadurch gekennzeichnet, dass** die Aufeinanderfolge der Schritte des Absaugens, Entleerens, Desolvierens und Brennens wiederholt wird.

6. Verfahren nach einem der vorstehenden Ansprüche, **dadurch gekennzeichnet, dass** beim Vorbereitungsschritt der Innenoberfläche der in das interne Röhrchen eingeführte Draht ein Strang in der Art von Diamant-Wolfram ist.

7. Verfahren nach einem der vorstehenden Ansprüche, **dadurch gekennzeichnet, dass** der Vorbereitungsschritt der Innenoberfläche auch durch einen chemischen Angriff realisiert wird.

8. Verfahren nach dem vorstehenden Anspruch, **dadurch gekennzeichnet, dass** der chemische Angriff des Vorbereitungsschritts der Innenoberfläche durch internes Besprengen mit Flüssigkeit oder Injektion von Dampf realisiert wird.

9. Verfahren nach einem der vorstehenden Ansprüche, **dadurch gekennzeichnet, dass** das Brennen der Beschichtung bei einer Temperatur realisiert wird, die zwischen 150°C und 450°C enthalten ist.

## Claims

1. Method for applying a fluorinated and/or ceramic, coloured coating on an internal wall of a low-diameter tube, such as a syringe needle, comprising the steps of:
- suction and/or flow, in the tube, of a liquid containing the coating;
- draining the liquid contained in the tube, at the end of which a coating deposit is obtained on the internal wall of the tube;
- dissolving the coating by forced air circulation;
- curing the coating;
**characterised in that** it comprises a step of preparing the internal surface carried out by mechanical attack, the step of preparing the internal surface being carried out using a wire introduced in the internal tube and driven by a movement back and forth against the internal wall of the tube.

2. Method according to claim 1, **characterised in that** the suction step is repeated a plurality of times before the drainage step.

3. Method according to claim 1, **characterised in that** the forced air circulation is carried out at a temperature between that of the ambient air and 150°C.

4. Method according to claim 1, **characterised in that** the forced air circulation is carried out in an oven.

5. Method according to any one of claims 1 to 3, carried out in that the sequence of suction, drainage, dissolving and curing steps is repeated

6. Method according to any one of the preceding claims, **characterised in that** during the step of preparing the internal surface, the wire introduced into the internal tube is a tungsten diamond filament.

7. Method according to any one of the preceding claims, **characterised in that** the step of preparing the internal surface is also carried out by chemical attack.

8. Method according to the preceding claim, **characterised in that** the chemical attack of the step of preparing the internal surface is carried out by internal spraying of liquid or injection of vapour.

9. Method according to any one of the preceding claims, **characterised in that** the curing of the coating is carried out at a temperature between 150°C and 450°C.
